# EUROPEAN PATENT SPECIFICATION

(11) **EP 0 512 359 B1**
(45) Date of publication and mention of the grant of the patent: **18.12.1996**
(21) Application number: 92107182.5
(22) Date of filing: 28.04.1992
(51) Int. Cl.: A61M 29/02

(54) **An angioplasty catheter and a method of producing it**
Angioplastie-Katheter und dessen Herstellungsverfahren
Cathéter pour angioplastie et procédé de fabrication

(30) Priority: 06.05.1991 IT TO910336
(43) Date of publication of application: 11.11.1992
(73) Proprietor: MINISTERO DELL' UNIVERSITA' E DELLA RICERCA SCIENTIFICA E TECNOLOGICA, 00144 Roma (IT)
(72) Inventor: De Rossi, Danilo, I-56017 San Giuliano Terme (Pisa) (IT); Ercolani, Mauro, I-00153 Roma (IT); Fenn, Maurizio, I-50046 Poggio A Caiano (Firenze) (IT); Venturelli, Luigi, I-25062 Concesio(Brescia) (IT)
(74) Representative: Bosotti, Luciano

(56) References cited:
- EP-A- 0 397 459
- WO-A-91/19529
- US-A- 4 406 656
- US-A- 5 032 113

## Description

### FIELD OF THE INVENTION

The present invention relates to a catheter of the type including a first tubular element extending within a second, coaxial tubular element with which it defines a space.

More particularly, the present invention relates to an angioplasty catheter which is used in cardiovascular treatment for performing so-called percutaneous transluminal coronary angioplasty or PTCA.

The invention also relates to a method for the continuous production of the angioplasty catheter.

As is known, in PTCA coronary angioplasty treatment, a catheter with an expandable portion is used to reopen a blood vessel which is partially or entirely blocked by plaques of atherosclerotic origin, that is, so-called stenoses.

In therapeutic angioplasty treatment, the catheter is first introduced into a femoral artery of the patient and is then passed along the artery until it reaches the stenotic blockage.

Stenotic deposits - which generally include lipids, complex carbohydrates, blood clots, fibrous tissues and sometimes deposits of calcium - are then pushed against the internal walls of the blood vessel by the expansion of the expandable portion of the catheter, generally by means of a fluid under pressure.

The various stages of the angioplasty treatment are generally monitored by radiographic equipment after a suitable contrasting liquid has been introduced into the blocked blood vessel.

### DESCRIPTION OF THE PRIOR ART

A first type of catheter of the prior art of the sector comprises the so-called balloon catheter which has an outer tubular element on which is formed, or to which is applied, a further tubular portion or balloon which can be expanded by the admission of a suitable pressurised fluid to the tubular element.

Thus, for example, the patent US-A-4 323 071 describes a coronary angioplasty device including a guide catheter in which a second expansion catheter, on which an annular expandable portion is formed, is guided for sliding. The expandable portion is formed by the hot plastic deformation of the outer wall of the expansion catheter to form an inflatable balloon.

In the rest condition, the balloon is folded onto itself to form a C-shaped cup.

A catheter of this type - that is, with a balloon fitted outside the catheter or formed by the plastic deformation of its outer wall - however, has a series of disadvantages which limit its practical application.

In fact, during the insertion and withdrawal of the catheter through the femoral artery, the outer wall of the balloon becomes puckered, making the catheter substantially more difficult to use. There is consequently a high risk of tearing or abrasion of the internal walls of the artery under treatment.

A second disadvantage of balloon catheters results from the fact that their return to the original condition, as a result of their being emptied, sometimes involves an undesirable bending of the catheter which contributes to the difficulty of removing it from the blood vessel, increasing the risk of injury to the patient being treated.

In order to overcome the disadvantages mentioned above, so-called "zero-profile" catheters, that is, catheters without portions applied or otherwise formed outside the catheter itself, have been proposed and produced according to the prior art of the sector.

Thus, for example, the patent US-A-4 706 670 describes a catheter including two coaxial, tubular elements which are joined at one end and form a space for receiving a suitable expansion fluid under pressure.

Of these tubular elements, the outer one - which is made of plastics material - is reinforced with continuous filaments forming a braid wound at an angle which varies from one region of the catheter to another.

More particularly, where the angle of winding is below a critical value of 54.73 degrees, the delivery of pressurised fluid into the space causes the outer tubular element to expand and form a swelling which is intended to act on the stenotic deposits with an action similar to that of a balloon. Where the angle at which the braid is wound is above the critical value, on the other hand, the introduction of the pressurised fluid causes the wall of the outer tubular element to contract.

Although, on the one hand, such a catheter overcomes the problems resulting from the presence of the balloon, on the other hand, it considerably complicates the structure of the catheter, the production of which therefore takes a long time, is expensive, and is not easy to control from a qualitative point of view. This results in the production of a considerable number of defective catheters which cannot be used.

The patent US-A-4 888 146 describes solutions intended preferably for the production of a catheter having two portions with different flexibility characteristics, with the interposition of a portion in which the two materials are mixed. The field of application of this technique is mainly that of single-cavity catheters for the injection of fluids. The document in question does not refer to the production of zero-profile balloons.

Application GB-A-2 191 145 describes a method and equipment for extruding a tube with lateral strips of different materials.

Application EP-A-0 377 749 provides for the guiding of the tip of a catheter in correspondence with branches in vessels in order to reach the desired expansion site more easily.

PCT Application W090/01302 relates to materials from which angioplasty catheters are made; there is reference neither to differentiation of the material along the catheter nor to the formation of zero-profile balloons.

Other documents of a certain interest are EP-A-0 277 368, EP-A-0 144 629, EP-A-0 346 012 and EP-A-0 354 695, as well as Japanese application 21 84 419. All of these documents describe catheters formed by two materials of different flexibilities, either by coextrusion or by successive extrusions. These relate essentially to the same field of application as US-A-4 888 146 and similar considerations therefore apply.

### OBJECTS AND SUMMARY OF THE INVENTION

More specifically, the present invention relates to an angioplasty catheter according to the preamble of Claim 1, which is known, e.g. from EP-A-0 408 198.

The technical problem on which the present invention is based is to provide a zero-profile angioplasty catheter which does not have the disadvantages complained of with reference to the prior art cited and which, at the same time, can be produced easily and quickly and with effective control from both quantitive and qualitative points of view.

According to the invention, this problem is solved by a catheter of the type indicated above having the further features set forth in Claim 1.

The invention also relates to the respective method of production, as set forth in Claim 13.

### DETAILED DESCRIPTION OF THE INVENTION

Further characteristics and the advantages of the catheter according to the invention will become clearer from the following detailed description of some embodiments thereof, given by way of non-limiting example with reference to the appended drawings 1-13, in which:
Figure 1 shows schematically and in plan an expansion unit including an angioplasty catheter according to the present invention,
Figure 2 is a longitudinal section of a detail of the catheter shown in Figure 1 taken on the lines III-III of Figure 3 and Figure 4, on an enlarged scale,
Figure 3 is a transverse section of the catheter of Figure 1 taken on the line I-I of Figure 2,
Figure 4 is a transverse section of the catheter of Figure 1, taken on the line II-II of Figure 2,
Figure 5 shows the detail of Figure 2 in an operative condition of the catheter of the present invention,
Figure 6 shows schematically and in plan a second embodiment of the expansion unit and of the catheter of the present invention,
Figure 7 is a longitudinal section of a detail of the catheter of Figure 6, taken on the lines IV-IV of Figures 8 and 9, on an enlarged scale,
Figure 8 is a transverse section of the catheter of Figure 6 taken on the line V-V of Figure 7,
Figure 9 is a transverse section of the catheter of Figure 6 taken on the line VI-VI of Figure 7,
Figures 10, 11 and 12 are transverse sections of the catheter of Figure 6 in three operative conditions,
Figure 13 is a perspective view of a further embodiment of the catheter of the present invention,
Figure 14 is an exploded, perspective view of an extrusion head for the continuous production of the catheter of the present invention,
Figure 15 is a longitudinal section of the extrusion head of Figure 14, on an enlarged scale, and
Figures 16 and 17 are longitudinal sections of two details of the extrusion head 14 on a further enlarged scale, in two operative conditions.

With reference to the drawings, an expansion unit for carrying out PTCA coronary angioplasty treatment is generally indicated 1.

The unit 1 includes a syringe 2 of wholly conventional type for delivering a suitable expansion fluid to a catheter 5 through a tube 3, by means of a collector-distributor 4.

The syringe 2 is connected, also conventionally, by means of a T-piece 6 and a tube 7, to a manometer 8 for monitoring the delivery pressure of the expansion fluid.

The unit 1 also includes a guide thread 9 on which the catheter 5 is intended to be mounted for sliding.

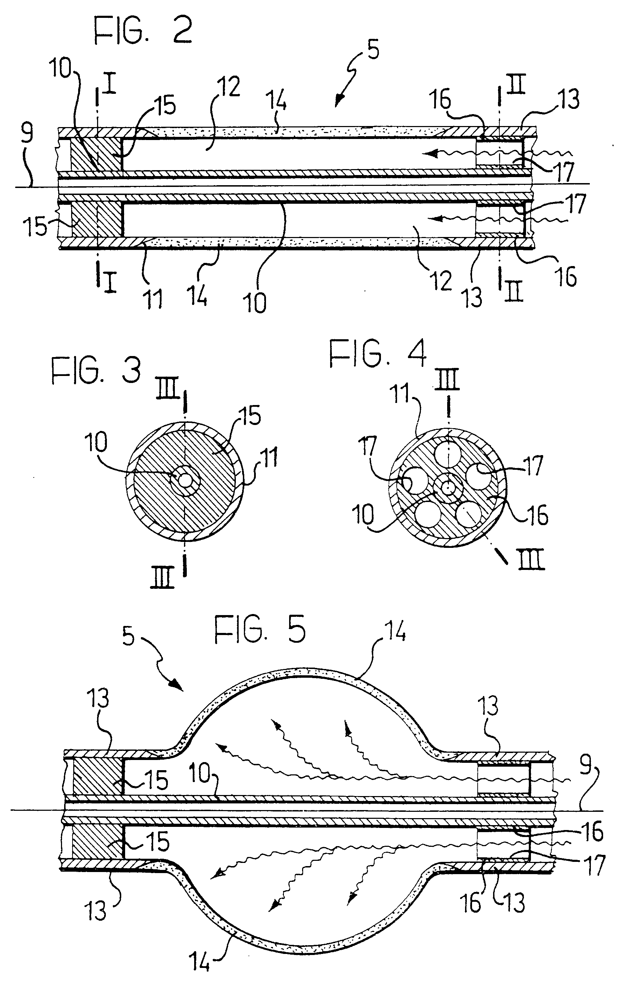

The catheter 5 (Fig. 2) includes a first tubular element 10 for housing the guide thread 9, extending coaxially within a second tubular element 11 with which it defines a space 12 of annular cross-section.

The outside diameter of the tubular element 11 is between 5 and 9 frenches (1.65 - 2.97 mm), preferably 7 frenches (2.31 mm), and the outside diameter of the tubular element 10 may vary between 2.5 and 5 frenches (0.8 - 1.65 mm), and is preferably 4 frenches (1.32 mm).

The second tubular element 11 includes a longer portion 13 which is made of a substantially rigid plastics material with a predetermined elastic modulus and a portion 14 which is made of a second, resiliently deformable plastics material having an elastic modulus lower than that of the first plastics material.

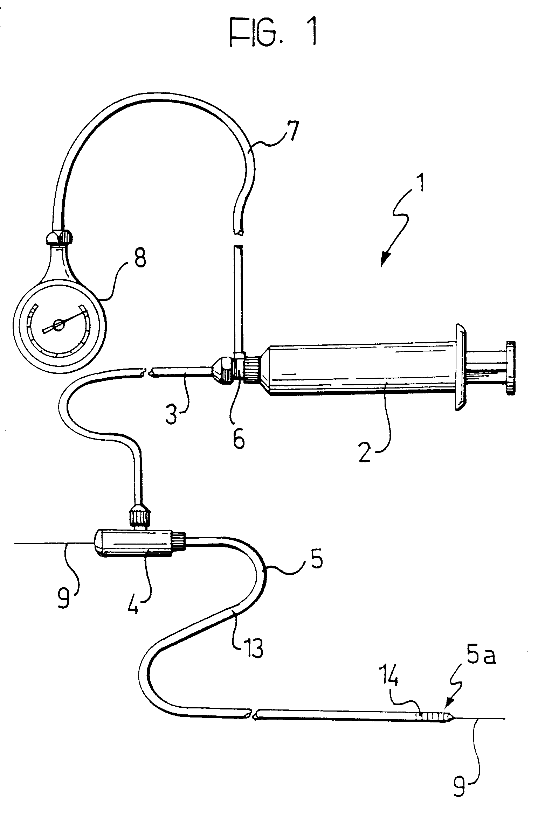

According to a preferred embodiment of the present invention, the resiliently deformable portion 14 is near the far end 5a of the catheter 5 from the collector-distributor 4 (see Figure 1).

The length of the portion 14 may vary between 3 and 7 mm and is preferably 5 mm.

The plastics material forming the rigid portion 13 of the catheter 5 may also be used to form the inner tubular element 10 and is constituted by any thermoplastic polymer which is biocompatible, that is, which has characteristics suitable for medical use. For this purpose, polyethylene, polypropylene, PVC, polyamide 1,2 or other polymers which can easily be selected by an expert in the art may be used.

According to one aspect of the present invention, the rigid portion 13 may be formed with the use of a thermoplastic elastomer, for example, a polyether-polyester, polyether-polyamide or styrene-diene block copolymer or a thermoplastic polyurethane.

The mechanical characteristics of the thermoplastic polymers or thermoplastic elastomers suitable for producing the portion 13 of the catheter of the present invention are given in Table 1 below.

**TABLE 1**

| MECHANICAL CHARACTERISTICS | MIN. VALUE | MAX. VALUE |
|---|---|---|
| Extensibility (Elongation at break) [%] ASTM D 638 | 200 | 500 |
| Breaking load (Tensile strength at break) [MPa] ASTM D 638 | 35 | 60 |
| Hardness ASTM D 22 40 | 78 SHORE A | 55 SHORE D |
| Load to cause 10% extension (elongation) [MPa] ASTM D 638 test 4 150 R37, BS 903-A 2-1971 | 3.9 | 14.2 |
| Young's Modulus (Modulus of elasticity) [MPa] ASTM D 638 | 14.6 | 145 |

The resiliently deformable plastics material constituting the portion 14 of the catheter 5, however, consists of any thermoplastic elastomer which has characteristics suitable for medical use and can be joined chemically to the polymer constituting the rigid portion 13 of the catheter. Thermoplastic elastomers usable include, for example, the same block copolymers as those which can be used to produce the rigid portion 13, such as polyether-polyester, polyether-polyamide, or styrene-diene, and the thermoplastic polyurethanes.

Of these, polyether-polyester and polyether-polyamide copolymers are preferred.

The mechanical characteristics of the thermoplastic elastomers usable to produce the resiliently deformable portion 14 are given in Table 2 below.

**TABLE 2**

| MECHANICAL CHARACTERISTICS | MIN. VALUE | MAX. VALUE |
|---|---|---|
| Young's modulus (Modulus of elasticity) [MPa) ASTM D 412 - loading rate 5.8 cm/min. | 3.50 | 7.30 |
| Extensibility (Elongation at break) [%] ASTM D 638 | 400 | 800 |
| Hardness ASTM D 2240 | 30 SHORE A | 65 SHORE A |
| Breaking load (Tensile strength at break) [MPa] ASTM D 638 | no less than 20 | |

As regards the load to cause 100% extension (elongation), the value of this parameter for the thermoplastic elastomers usable must be less than those mentioned in Table 1 with reference to the materials usable to produce the substantially rigid portion of the catheter.

The tubular elements 10 and 11 are interconnected by an annular partition 15 which is fixed in the space 12 at the far end of the portion 14 from the collector-distributor 4.

The partition 15 thus closes the space 12 at that end of the portion 14.

In order to increase the rigidity of the catheter 5, it also has a reinforcing ring 16 with a plurality of parallel axial holes 17 (see Figures 2 and 4) at the end of the portion 14 nearest the collector-distributor 4.

The methods of carrying out PTCA therapeutic angioplasty treatment by means of the catheter 4 of the present invention will now be described with reference to Figures 1 and 5.

A first step of the angioplasty treatment provides for the insertion of the guide thread 9 into the cavity of a peripheral blood vessel of a patient, for example, one of the two femoral arteries. This is achieved by wholly conventional techniques known in the field.

The guide thread 9 is then passed through the blood vessel until it reaches the stenotic blockages present, for example, at the coronary level.

The correct positioning of the guide thread and the subsequent steps of the angioplasty treatment can be followed by wholly conventional radiographic techniques after a suitable contrasting liquid has been introduced into the coronary artery.

Once the guide thread 9 has been positioned, the catheter 5 is introduced into the blood vessel in turn and positioned in correspondence with the stenotic blockages after sliding along the guide thread 9 already positioned in the blood vessel.

During its sliding, the catheter 5 is in the configuration shown in Figure 2 and offers minimal resistance - by virtue of its "zero-profile" characteristics - as it advances progressively through the patient's arteries.

Once it has reached the optimal position to start angioplasty treatment of the stenoses, that is, when the resiliently deformable portion 14 of the catheter 5 is facing the stenoses, a suitable expansion fluid is introduced into the space 12 under pressure by means of the syringe 2. The pressure imparted to the fluid by the syringe is monitored by the manometer 8 and, once the space 12 is filled, causes the resiliently deformable portion 14 to expand. The catheter thus assumes the configuration shown in Figure 5.

The stenotic deposits are thus pushed against the inner wall of the coronary artery by the portion 14 and kept there under pressure for a predetermined period of time.

The pressure of the expansion fluid monitored by the manometer 8 is preferably between 3 and 5 atmospheres and is kept between those values for about 10 seconds. This prevents interruption of the blood circulation from injuring the patient being treated.

Naturally, if the vessel has not been reopened in a satisfactory manner at the end of this period of compression of the stenotic deposits, the expansion of the resiliently deformable portion 14 of the catheter may be repeated in a similar manner until the desired results are achieved.

If more stenotic blockages are present in the coronary artery, the catheter 5 may be pushed further along the artery and positioned in correspondence with another stenosis.

The blood vessel is then reopened in exactly the same way as described above.

Once the angioplasty treatment of the stenosis or stenoses is complete, the catheter and the guide thread 9 are withdrawn from the blood vessel and removed from the patient.

To advantage, since the catheter 5 of the present invention is of the so-called "zero-profile" type, that is, the type in which its portion which can be expanded like a balloon - in this case, the portion 14 of the tubular element 11 - has the same outside diameter as the rest of the catheter, it can be removed from the blood vessel easily without the risk of injury or laceration of the tissues of the vessel.

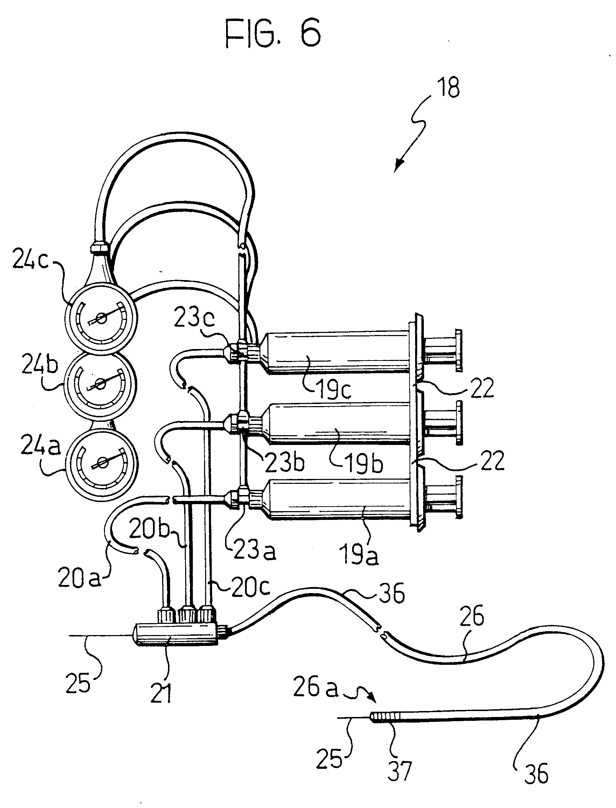

A second embodiment of the catheter of the present invention will now be described with reference to Figures 6 to 12.

With reference to these drawings, an expansion unit, generally indicated 18, includes three syringes 19a, 19b and 19c of conventional type connected by respective tubes 20a, 20b and 20c to a collector-distributor 21. The syringes are fixed together by a plate-like connecting element 22.

The syringes 19a, 19b and 19c are also connected to corresponding manometers 24a, 24b and 24c, in conventional manner, by respective T-pieces 23a, 23b and 23c.

The expansion unit 18 also includes a guide thread 25 which can be fixed to the collector-distributor 21 and on which a catheter 26 is mounted slidably and removably.

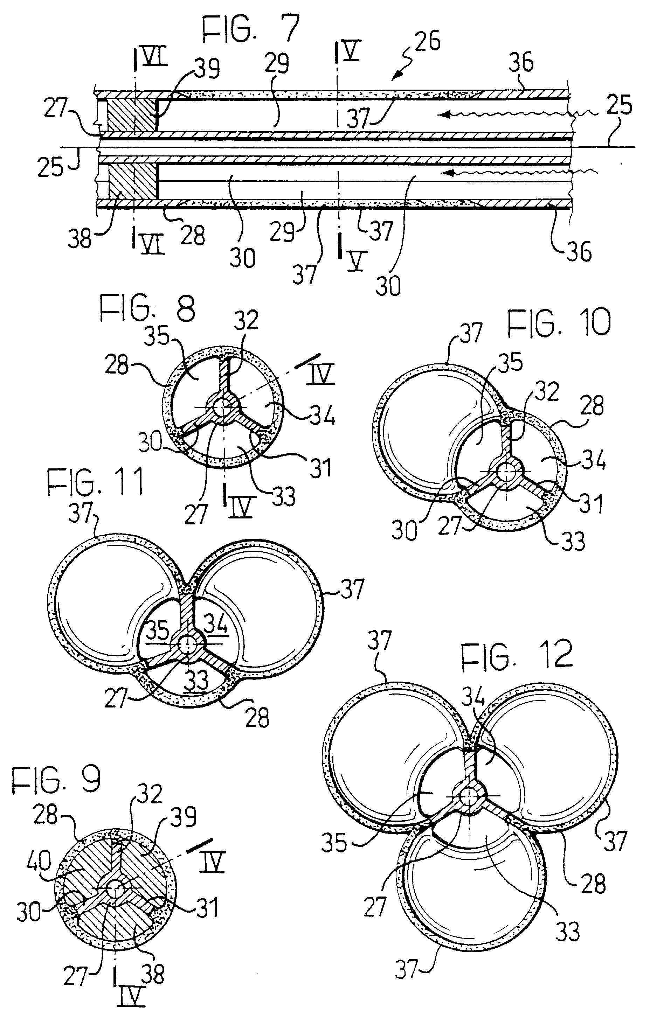

The catheter 26 includes a first tubular element 27 for housing the guide thread 25, extending coaxially within a second tubular element 28 with which it forms a space 29 of annular cross-section (see Figure 7).

With reference to Figures 8 and 9, it should be noted that the tubular elements 27 and 28 are connected throughout the length of the catheter 26 by three ribs 30, 31 and 32 which extend radially from the tubular element 27 and are inclined to each other at angles of about 120 degrees. Three adjacent chambers 33, 34 and 35, hermetically sealed from one another, are thus defined in the space 29.

The tubular elements 27 and 28 are thus connected without breaks of continuity throughout the length of the catheter 26.

As described above for the catheter 5, the tubular element 28 has a longer portion 36 which is made of a substantially rigid plastics material with a predetermined elastic modulus and a portion 37 which is incorporated in the preceding portion 36 without breaks in continuity and is made of a second, resiliently deformable plastics material with a lower elastic modulus than the portion 36 (see Figure 6).

In this embodiment, the portion 37 which is made of resiliently deformable plastics material is also preferably formed near the end 26a of the catheter 26 furthest from the collector-distributor 21 (see Figure 6).

The catheter 26 also includes three partitions 38, 39 and 40 which are fixed in the chambers 33, 34 and 35 at the end of the portion 37 furthest from the collector-distributor 21 (see Figure 7).

To advantage, the ribs 30-32 which define the three chambers 33-35 in the space 29 substantially increase the rigidity of the catheter 26 which is consequently easier to use and more reliable.

The method of carrying out a PTCA therapeutic angioplasty treatment by means of the catheter 26 of the present invention will now be described with reference to Figures 7 to 12.

The steps of inserting the guide thread 25 and the catheter 26 into a blood vessel and positioning them correctly therein - that is - in correspondence with the stenotic blockages - are carried out in the manner already described above.

Once the resiliently-deformable portion 37 of the catheter 26 is positioned in correspondence with a stenosis, the stenosis is compressed against the internal wall of the coronary artery by the expansion of the portion 37 by means of a suitable expansion fluid under pressure.

The expansion is effected by the injection of the fluid into each of the chambers 33-35 by means of the syringes 19a-19c. The pressure in each chamber is monitored by the manometers 24a-24c.

With reference to Figure 12, it should be noted that the chambers 33-35 expand in directions spaced by angles of approximately 120 degrees and give the catheter a characteristic three-lobed configuration.

To advantage, the catheter 26 thus enables the stenoses to be treated by angioplasty without interruption of the blood-flow through the artery. By virtue of the particular "lobed" structure of the catheter, the cavity of the vessel is not in fact completely obstructed during the compression of the stenotic deposits and the compression can therefore be prolonged beyond the maximum limit of 10 seconds allowed by the catheters of the prior art.

To advantage, this substantially reduces the laboriousness of the angioplasty treatment and reduces the risk to the patient.

The particular structure of the catheter 26 also has the advantage that it enables the stenotic deposits to be compressed selectively by the admission of the expansion fluid to the most suitable of the chambers 33, 34 and 35.

According to the number of chambers supplied with the expansion fluid, the catheter 26 will assume the configurations shown in Figures 10, 11 and 12, respectively.

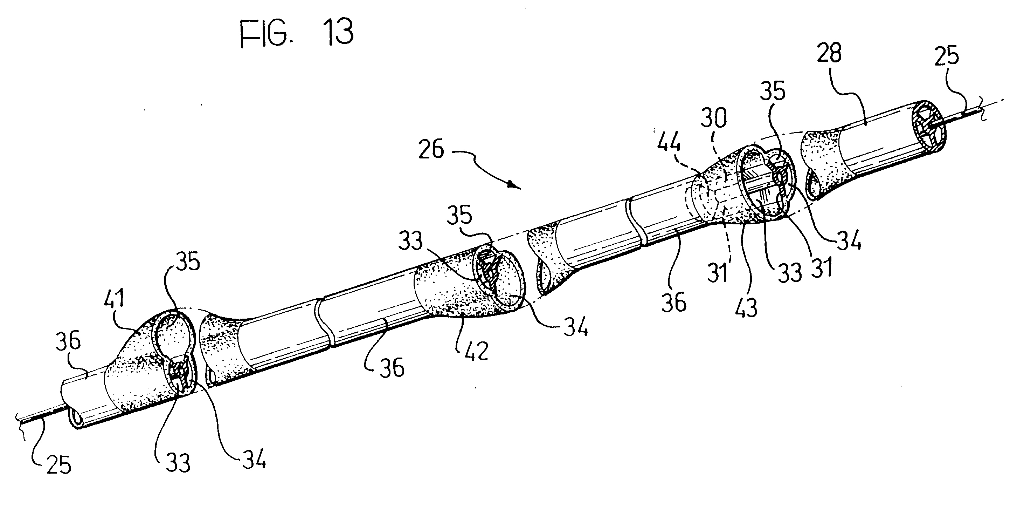

A further embodiment of the catheter of the present invention, in which the catheter 26 includes three portions 41, 42 and 43 made of a resiliently-deformable plastics material and incorporated in the rigid portion 36 of the tubular element 28 at predetermined intervals will now be described with reference to Figure 13.

In this further embodiment of the invention, each of the chambers 33-35 is closed by its own partition at one end of one of the portions 41-43.

More particularly, the chamber 33 is closed by a partition 44 at one end of the portion 41 and, similarly, the remaining chambers 34 and 35 are closed by respective partitions, not visible in Figure 13, at the ends of the portions 42 and 43 remote from the collector-distributor 21.

This further embodiment of the invention offers the advantage that several stenotic deposits positioned in angularly offset positions along the blood vessel can be compressed simultaneously whilst, at the same time, the bloodflow through the artery is safeguarded during the angioplasty treatment.

In any case, the catheter 26 of the present invention enables easier and quicker treatment of stenotic deposits located at various points along the coronary artery.

The present invention also relates to a method of producing the outer tubular element of the catheter according to the invention, the method providing for the preliminary steps of fluidising the first and second plastics materials separately and including basically the successive steps of:
a) supplying the first plastics material in the fluid state to an extrusion path defined in a die so as to extrude continuously a substantially rigid portion of the second tubular element,
b) stopping the supply of the first plastics material and simultaneously supplying the second plastics material to the extrusion path, in the fluid state,
c) completing the extrusion of the first plastics material remaining in the extrusion path by means of the thrust of the second plastics material and simultaneously joining the plastics materials together,
d) extruding the second plastics material along the path to produce a resiliently deformable portion of the second tubular element,
e) stopping the supply of the second plastics material to the extrusion path and simultaneously re-establishing the supply of the first plastics material,
f) completing the extrusion of the second plastics material remaining in the extrusion path by means of the thrust of the first plastics material and simultaneously joining the plastics materials together,
g) extruding the first plastics material along the extrusion path to produce continuously the substantially rigid portion of the second tubular element.

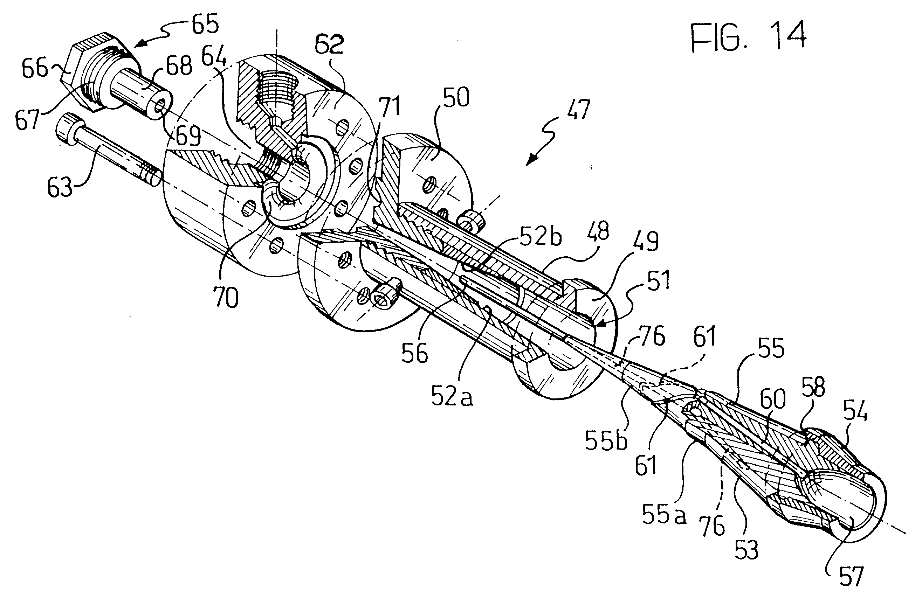

In particular the extrusion head shown in Figures 14-17 can be used to carry out the method.

With reference to the drawings, an extrusion head, generally indicated 47, is mounted downstream of a plasticising screw of an extruder which is not shown since it is wholly conventional. The head 47 includes a cylindrical block 62 and a cylindrical body 48 with flanges 49 and 50 at its opposite ends.

The head 47 also has an axial through-hole 51 defining a seat 52 for housing a pin 53 with form coupling. The seat 52 has a first, cylindrical portion 52a and a second, substantially conical portion 52b.

The pin 53 in turn has a head portion 54, a central body 55, and a rod 56.

The head portion 54, which has a central, axial recess 57, has an annular shoulder 58 for cooperating in abutment with the flange 49 to position the pin 53 correctly in the seat 52.

The central body 55 of the pin 53, however, has two portions 55a and 55b which are substantially cylindrical and conical, respectively, and are intended to mate with the portions 52a and 52b of the seat 52.

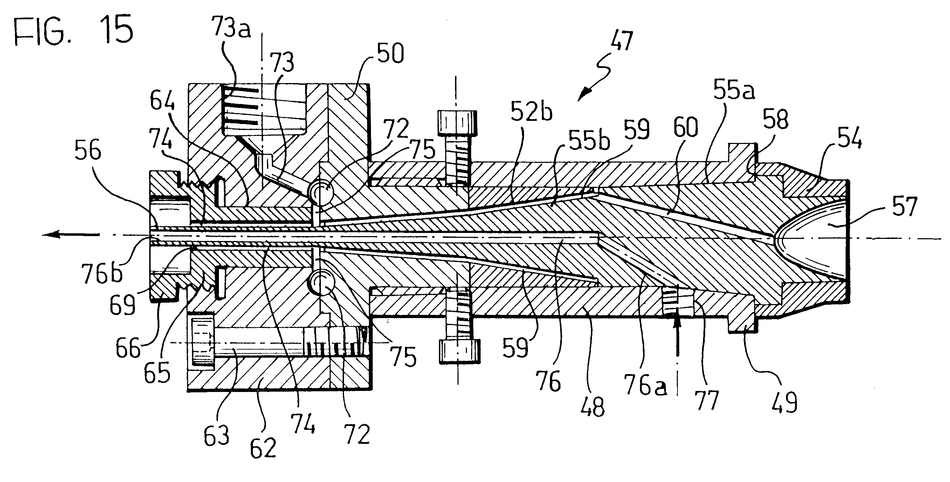

More precisely, the pin and the seat are coupled so as to define an annular space 59 between the substantially conical portions 52b of the seat 52 and 55b of the body 55 (Fig. 15).

A duct 60 extending longitudinally and transversely within the central body 55 of the pin 53 connects the recess 57 to an external groove 61 extending in a spiral around the portion 55b of the pin 53.

With reference to Figure 15, it should be noted that, when the pin 53 is mounted in its seat 52, the space 59 is connected to the recess 57 by the duct 60.

The pin 53 includes a further duct 76 which is in fluid communication, through a hole 77 in the cylindrical body 48, with an external source, not shown, of a suitable pressurised fluid, for example air.

The duct 76 (see Figure 15) includes a first portion 76a which extends transversely through the portion 55a of the pin 53 and a second portion 76b which extends coaxially with the pin through the portion 55b of the body 55 and through the rod 56.

The block 62 of the extrusion head 47 is fixed to the flange 50 of the cylindrical body 48 by wholly conventional screws 63 and has a central extrusion hole 64 extending axially from the space 59.

A die 65 is mounted in the hole 64, for example, by a threaded coupling.

The die includes an integral head 66 formed like a hexagonal nut, a threaded portion 67 and a tubular body 68, and has an axial hole 69.

With reference to Figure 15, it should also be noted that the hole 69 and the rod 56 of the pin 53 define in the die 65 an extrusion path 74 which extends axially from the annular space 59 and is connected to the plasticiser screw by the duct 60 and the recess 57.

With reference to Figures 14 and 15, it can be seen that the block 62 and the flange 50 have respective facing annular grooves 70 and 71 which define a toroidal chamber 72 coaxially surrounding the extrusion path 74 in the extrusion head 47.

The toroidal chamber 72 is connected by a duct 73 extending transverse the extrusion hole 64 to a plasticiser screw of a second extruder which is not shown since it is wholly conventional.

The duct 73 has a threaded opening 73a for connection to the extruder.

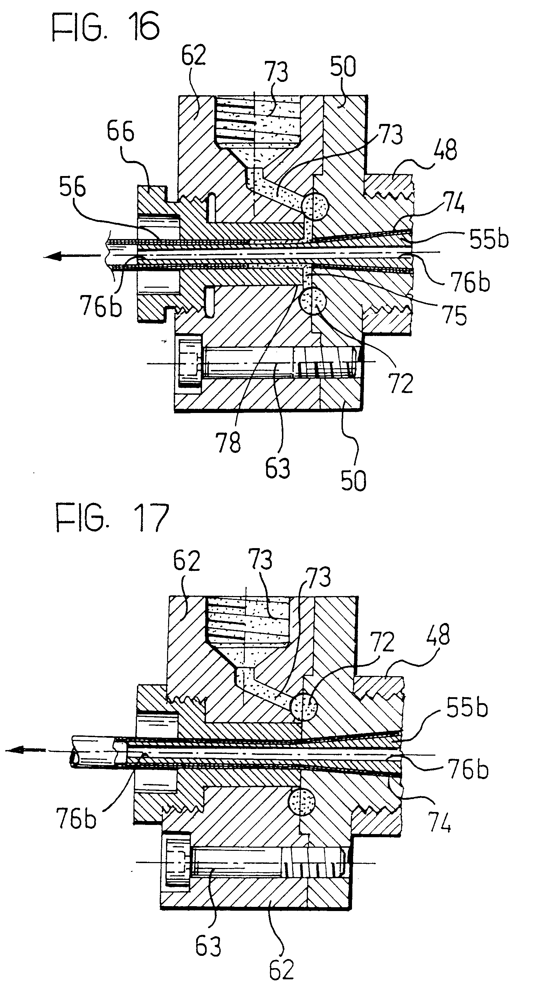

With reference to Figure 15, it should also be noted that, by virtue of the presence of the threaded portion 67, the die 65 is movable axially in the extrusion hole 64 between a first position in which it cooperates with the flange 50 to define an annular duct 75 extending radially between the toroidal chamber 72 and the extrusion path 74, and a second position in which the annular duct 75 is completely shut off (see Figures 16 and 17).

The extrusion path 74 is thus connected to the second plasticising screw by the annular duct 75, the toroidal chamber 72 and the duct 73.

According to a characteristic of the present invention, the extrusion path 74 can be disconnected from the plasticising screw, since the die 65 constitutes a means 78 for cutting off the flow of plastics material coming from the duct 73.

The method of the present invention provides for the first, substantially rigid plastics material, for example, polyethylene, and the second, flexible plastics material, for example, a polyether-polyamide thermoplastic elastomer known commercially by the name PEBAX, to be plasticised separately by conventional extrusion screws.

During the plasticising operations, the plastics materials reach temperatures of the order of 200-250 degrees C, which are more than sufficient to transform them into highly viscous fluids.

According to the method of the present invention, the first, rigid plastics material, in this case, polyethylene, is supplied by the extrusion screw to the recess 57 in the head portion 54 of the pin 53.

The fluid plastics material is then urged through the duct 60 and the annular space 59, in which it is distributed uniformly by virtue of the grooves 61.

As a result of the thrust of the extrusion screw, the polyethylene then reaches the extrusion path 74 defined in the die 65 which imparts the desired tubular shape to the plastics material.

During this stage of the method, the die is in the position shown in Figure 17, that is, in a position such as completely to shut off the annular duct 75.

The substantially rigid portion 13 or 36 of the outer tubular element of the catheter of the present invention is thus extruded continuously from the head 47.

Once the portion made of polyethylene has reached a predetermined length, according to the method of the present invention, the following steps are carried out simultaneously:
- the screw for extruding the first plastics material, polyethylene, is stopped, thus stopping the supply of polyethylene to the extrusion path 74,
- the screw for extruding the thermoplastic elastomer, PEBAX, is started, introducing the PEBAX into the duct 73 and the toroidal chamber 72, and
- the die 65 is moved away from the flange 50 of the head 47 in the extrusion hole 64, opening the annular duct 75 between the toroidal chamber 72 and the extrusion path 74.

The die is moved in the extrusion hole 64 simply by being unscrewed, for example, by a mechanical arm acting on the hexagonal nut-like head 66.

As a result of these steps, the die 65 is in the position shown in Figure 16, thus enabling the thermoplastic elastomer to be supplied from the duct 73 to the extrusion path 74.

The resiliently deformable plastics material continues to be extruded from the die without a break in continuity; in fact, the thermoplastic elastomer which is urged into the extrusion path 74 completes the extrusion of the polyethylene, to which it is joined chemically. It is then extruded to form the resiliently deformable portion 14 or 37 of the outer tubular element of the catheter 5.

Once the resiliently deformable portion has reached the desired length, according to the method of the present invention, the following steps are carried out simultaneously:
- the screw for extruding the thermoplastic elastomer is stopped, thus stopping the supply of the thermoplastic elastomer to the duct 73,
- the screw for extruding the polyethylene is restarted, re-establishing the flow of polyethylene through the space 59 and, at the same time, supplying it to the extrusion path 74,
- the die 65 is moved towards the flange 50 of the head 47, cutting off the flow of thermoplastic elastomer and closing the annular duct 75.

As a result of these steps, the extrusion of the polyethylene is re-established, producing a new substantially rigid portion of the outer tubular element of the catheter.

As already described with reference to the thermoplastic elastomer, the flow of polyethylene into the extrusion path 74 completes the extrusion of the thermoplastic elastomer, carrying out the so-called "washing" of the extrusion path.

In this case also, the chemical compatibility of the polyethylene and the thermoplastic elastomer enables, for example, the resiliently deformable portion 14 to be joined chemically to the rigid portion 13 of the tubular element 11.

Throughout all the aforementioned steps, a continuous flow of pressurised air is supplied to the duct 76 to impart the desired shape to the extruded tubular element.

The element then undergoes wholly conventional cooling and setting steps carried out by equipment which is also conventional and not shown.

Naturally, the steps of the method of the present invention may, to advantage, be carried out automatically, the operation of the extrusion screws, any heating or cooling means upstream and downstream of the extrusion head 47, the movement - by mechanical arms - of the die 65 in the head 47, etc. being controlled by a central control unit.

The method of the present invention also enables the lengths of the various rigid portions 13, 3^{b} and resiliently deformable portions 14, 37 of the outer tubular elements 11, 28 of the catheter to be adjusted continuously and in a quick and easy manner so that the desired number of portions can be inserted in the desired positions.

Once the outer tubular element has been produced as described and once the inner tubular element of the catheter has been extruded by conventional techniques - possibly with the use of the same head 47 - the catheter can finally be formed by the assembly of the two tubular elements, for example, by their connection by adhesives, with the use of the annular partition 14 and, in the case of the catheter 5, preferably also the perforated reinforcing ring 15.

Thus, the method of the present invention, to advantage, considerably simplifies the production of a "zero-profile" angioplasty catheter which can be produced continuously in an extremely quick and easy manner.

The outer tubular element 28 of the catheter 26 can be produced in a manner similar to that described above, and the chambers 33-35 can be formed in the space therein by suitable shaping of the internal tubular element 27 by means of a conventional die.

The tubular elements 27 and 28 can be assembled as described above with the use of adhesives or other known connecting techniques to produce the catheters shown in Figures 7 - 13.

## Claims

1. An angioplasty catheter of the type including a first tubular element (10, 27) extending within a second, coaxial tubular element (11, 28) with which it defines a space (12, 29), characterised in that the second tubular element (11, 28) includes, without breaks in its continuity, a portion (13, 36) made of a first, substantially rigid plastics material of a predetermined elastic modulus and at least one second portion (14, 37) made of a second, resiliently deformable plastics material which has an elastic modulus lower than that of the first plastics material and has been joined chemically thereto, the space (12, 29) being closed in correspondence with one end of the second portion (14, 37).

2. A catheter according to Claim 1, characterised in that the second plastics material is a thermoplastic elastomer.

3. A catheter according to Claim 2, characterised in that the thermoplastic elastomer is selected from the group including polyether-polyester, polyamide-polyether and butadiene-styrene block copolymers, and thermoplastic polyurethanes.

4. A catheter according to Claim 1, characterised in that the first plastics material is selected from the biocompatible thermoplastic polymers.

5. A catheter according to Claim 4, characterised in that the thermoplastic polymer is selected from the group including: polyethylene, polypropylene, PVC, polyamide 1, 2, and the thermoplastic elastomers.

6. A catheter according to Claim 5, characterised in that the first plastics material is a polyether-polyester block copolymer, and in that the second plastics material is polyamide 1, 2.

7. A catheter according to Claim 1, characterised in that the second, resiliently deformable portion (14, 37) of the second tubular element (11, 28) is between 3 and 7 mm long.

8. A catheter according to Claim 1, characterised in that it includes a ring (16) which has a plurality of coaxial holes and is disposed in the space (12) in correspondence with the end of the resiliently deformable portion (14) which is not closed by the wall (15).

9. A catheter according to Claim 1, characterised in that a plurality of ribs (30, 31, 32) extend radially in the space (29) between the tubular elements (27, 28) throughout the length of the space (29) and define a plurality of adjacent chambers (33, 34, 35) which are hermetically sealed from one another.

10. A catheter according to Claim 9, characterised in that it includes three ribs.

11. A catheter according to Claim 10, characterised in that the ribs which extend radially between the tubular elements (27, 28) are inclined to each other at angles of about 120 degrees.

12. A catheter according to Claim 9, characterised in that it includes at least two portions (41, 42, 43) of the resiliently deformable plastics material spaced at predetermined intervals, and in that the chambers (33, 34, 35) are closed by respective walls (38, 39, 40) each in correspondence with one end of a portion (41, 42, 43).

13. A method of producing an angioplasty catheter according to Claims 1-12, in which the second tubular element (28) is produced continuously and which includes the preliminary steps of fluidising the first and second plastics materials separately, characterised in that it includes the steps of:
a) supplying the first plastics material in the fluid state to an extrusion path defined in a die so as to extrude continuously a substantially rigid portion of the second tubular element,
b) stopping the supply of the first plastics material and simultaneously supplying the second plastics material to the path, in the fluid state,
c) completing the extrusion of the first plastics material remaining in the extrusion path by means of the thrust of the second plastics material and simultaneously joining the plastics materials together,
d) extruding the second plastics material along the path to produce a resiliently deformable portion of the second tubular element,
e) stopping the supply of the second plastics material to the extrusion path and simultaneously re-establishing the supply of the first plastics material,
f) completing the extrusion of the second plastics material remaining in the extrusion path by means of the thrust of the first plastics material and simultaneously joining the plastics materials together,
g) extruding the first plastics material along the extrusion path to produce a substantially rigid portion of the second tubular element.

## Patentansprüche

1. Angioplasie-Katheter mit einem ersten schlauchartigen Element (10, 27), das in einem zweiten, koaxialen schlauchartigen Element (11, 28) verläuft, mit dem es einen Zwischenraum (12, 29) bildet, dadurch gekennzeichnet, daß das zweite schlauchartige Element (11, 28) ohne Unterbrechung einen Teil (13, 36), der aus einem ersten, im wesentlichen steifen Kunststoff mit einem vorgegebenen Elastizitätsmodul besteht, sowie zumindest einen zweiten Teil (14, 37) aufweist, der aus einem zweiten elastisch verformbaren Kunststoff besteht, der einen Elastizitätsmodul besitzt, der kleiner als der Elastizitätsmodul des ersten Kunststoffs ist, und der chemisch mit dem ersten Kunststoff verbunden wurde, wobei der Zwischenraum (12, 29) in Übereinstimmung mit einem Ende des zweiten Teils (14, 37) verschlossen ist.

2. Katheter gemäß Anspruch 1, dadurch gekennzeichnet, daß der zweite Kunststoff ein thermoplastisches Elastomer ist.

3. Katheter gemäß Anspruch 2, dadurch gekennzeichnet, daß das thermoplastische Elastomer aus einer Gruppe ausgewählt wird, die Polyäther-Polyester, Polyamid-Polyäther und Butadien-Styrol Blockcopolymere sowie thermoplastische Polyurethane enthält.

4. Katheter gemäß Anspruch 1, dadurch gekennzeichnet, daß der erste Kunststoff aus bioverträglichen, thermoplastischen Polymeren ausgewählt wird.

5. Katheter gemäß Anspruch 4, dadurch gekennzeichnet, daß das thermoplastische Polymer aus einer Gruppe ausgewählt wird, die aufweist: Polyäthylen, Polypropylen, PVC, Polyamid 1, 2 sowie thermoplastische Elastomere.

6. Katheter gemäß Anspruch 5, dadurch gekennzeichnet, daß der erste Kunststoff ein Polyäther-Polyester Blockcopolymer ist, und daß der zweite Kunststoff ein Polyamid 1, 2 ist.

7. Katheter gemäß Anspruch 1, dadurch gekennzeichnet, daß der zweite elastisch verformbare Teil (14, 37) des zweiten schlauchartigen Elements (11, 28) zwischen 3 und 7mm lang ist.

8. Katheter gemäß Anspruch 1, dadurch gekennzeichnet, daß der Katheter einen Ring (16) aufweist, der eine Vielzahl von koaxialen Öffnungen besitzt und im Zwischenraum (12) in Übereinstimmung mit jenem Ende des elastisch verformbaren Teils (14) angeordnet ist, das nicht mit der Wand (15) verschlossen ist.

9. Katheter gemäß Anspruch 1, dadurch gekennzeichnet, daß eine Vielzahl von Rippen (30, 31, 32) im Zwischenraum (29) radial zwischen den schlauchartigen Elementen (27, 28) über die Länge des Zwischenraums (29) verläuft und eine Vielzahl von benachbarten Kammern (33, 34, 35) bildet, die voneinander hermetisch abgedichtet sind.

10. Katheter gemäß Anspruch 9, dadurch gekennzeichnet, daß der Katheter drei Rippen aufweist.

11. Katheter gemäß Anspruch 10, dadurch gekennzeichnet, daß die Rippen, die radial zwischen den schlauchartigen Elementen (27, 28) verlaufen, zueinander unter einem Winkel von etwa 120° geneigt sind.

12. Katheter gemäß Anspruch 9, dadurch gekennzeichnet, daß der Katheter zumindest zwei Teile (41, 42, 43) aus einem elastisch verformbaren Kunststoff aufweist, die in vorgegebenen Abständen beabstandet sind, und daß die Kammern (33, 34, 35) mit entsprechenden Wänden (38, 39, 40) verschlossen sind, von denen jede einem Ende eines Teils (41, 42, 43) entspricht.

13. Verfahren zur Herstellung eines Angioplasie-Katheters gemäß Anspruch 1-12, wobei das zweite schlauchartige Element (28) in einer Endlosfertigung hergestellt wird, wobei das Verfahren die vorbereitenden Schritte aufweist, um den ersten und zweiten Kunststoff getrennt fließend zu machen, dadurch gekennzeichnet, daß das Verfahren folgende Schritte aufweist:
a) Zuführen des ersten Kunststoffs im Fließzustand zu einer Preßstrecke, die in einer Preßform ausgebildet ist, um fortlaufend einen im wesentlichen starren Teil des zweiten schlauchartigen Elements zu pressen,
b) Anhalten der Zufuhr des ersten Kunststoffs und gleichzeitiges Zuführen des zweiten Kunststoffs im Fließzustand zur Strecke,
c) Beenden des Pressens des ersten Kunststoffs, der in der Preßstrecke übrig ist, mit Hilfe des Drucks des zweiten Kunststoffs und gleichzeitiges Verbinden der Kunststoffe miteinander,
d) Pressen des zweiten Kunststoffs längs der Strecke, um einen elastisch verformbaren Teil des zweiten schlauchartigen Elements zu erzeugen,
e) Anhalten der Zufuhr des zweiten Kunststoffs zur Preßstrecke und gleichzeitiges Wiederherstellen der Zufuhr des ersten Kunststoffs,
f) Beenden des Pressens des zweiten Kunststoffs, der in der Preßstrecke übrig ist, mit Hilfe des Drucks des ersten Kunststoffs und gleichzeitiges Verbinden der Kunststoffe miteinander,
g) Pressen des ersten Kunststoffs längs der Preßstrecke, um einen im wesentlichen starren Teil des zweiten schlauchartigen Elements zu erzeugen.

## Revendications

1. Cathéter pour angioplastie du type comprenant un premier élément tubulaire (10, 27) s'étendant à l'intérieur d'un second élément tubulaire coaxial (11, 28) avec lequel il définit un espace (12, 29), caractérisé en ce que le second élément tubulaire (11, 28) comprend, sans rupture dans sa continuité, une partie (13, 36) faite dans une première matière plastique sensiblement rigide, d'un module élastique prédéterminé, et au moins une seconde partie (14, 37) faite dans une seconde matière plastique élastiquement déformable ayant un module élastique inférieur à celui de la première matière plastique et qui est unie par voie chimique à celle-ci, l'espace (12, 29) étant fermé en correspondance avec une extrémité de la second partie (14, 37).

2. Cathéter selon la revendication 1, caractérisé en ce que la seconde matière plastique est un élastomère thermoplastique.

3. Cathéter selon la revendication 2, caractérisé en ce que l'élastomère thermoplastique est choisi dans le groupe comprenant des copolymères séquencés de polyester-polyéther, de polyéther-polyamide et de styrène-butadiène et des polyuréthannes thermoplastiques.

4. Cathéter selon la revendication 1, caractérisé en ce que la première matière plastique est choisie dans le groupe des polymères thermoplastiques biocompatibles.

5. Cathéter selon la revendication 4, caractérisé en ce que le polymère thermoplastique est choisi dans le groupe comprenant : le polyéthylène, le polypropylène, le PVC, le polyamide 1,2, et les élastomères thermoplastiques.

6. Cathéter selon la revendication 5, caractérisé en ce que la première matière plastique est un copolymère séquencé de polyester-polyéther et en ce que la deuxième matière plastique est le polyamide 1,2.

7. Cathéter selon la revendication 1, caractérisé en ce que la seconde partie élastiquement déformable (14, 37) du second élément tubulaire (11, 28) a une longueur comprise entre 3 et 7 mm.

8. Cathéter selon la revendication 1, caractérisé en ce qu'il comprend une bague (16) qui comporte plusieurs trous coaxiaux et qu'il est disposé dans l'espace (12) en correspondance avec l'extrémité de la partie élastiquement déformable (14) qui n'est pas fermée par la paroi (15).

9. Cathéter selon la revendication 1, caractérisé en ce que plusieurs nervures (30, 31, 32) s'étendent radialement dans l'espace (29) entre les éléments tubulaires (27, 28) sur toute la longueur de l'espace (29) et définissent plusieurs chambres adjacentes (33, 34, 35) qui sont scellées de manière hermétique les unes par rapport aux autres.

10. Cathéter selon la revendication 9, caractérisé en ce qu'il comprend trois nervures.

11. Cathéter selon la revendication 10, caractérisé en ce que les nervures qui s'étendent radialement entre les éléments tubulaires (27, 28) sont inclinées l'une par rapport à l'autre selon des angles d'environ 120 degrés.

12. Cathéter selon la revendication 9, caractérisé en ce qu'il comprend au moins deux parties (41, 42, 43) faites dans une matière plastique élastiquement déformable espacées par des intervalles prédéterminés, et en ce que les chambres (33, 34, 35) sont fermées par des parois respectives (38, 39, 40) chacune en correspondance avec une extrémité d'une partie (41, 42, 43).

13. Procédé de fabrication d'un cathéter pour angioplastie selon les revendications 1 à 12, dans lequel le second élément tubulaire (28) est fabriqué en continu, et qui comprend les étapes préliminaires consistant à fluidiser les première et seconde matières plastiques séparément, caractérisé en ce qu'il comprend les étapes consistant à :
(a) amener la première matière plastique à l'état fluide vers une voie d'extrusion définie dans une filière de manière à extruder en continu une partie sensiblement rigide du second élément tubulaire,
(b) suspendre l'amenée de la première matière plastique et amener simultanément la seconde matière plastique vers la voie, à l'état fluide,
(c) achever l'extrusion de la première matière plastique restant dans la voie d'extrusion par la poussée de la seconde matière plastique et en réunissant simultanément les matières plastiques,
(d) extruder la seconde matière plastique le long de la voie pour produire une partie élastiquement déformable du second élément tubulaire,
e) suspendre l'amenée de la seconde matière plastique vers la voie d'extrusion et rétablir simultanément l'amenée de la première matière plastique,
f) achever l'extrusion de la seconde matière plastique restant dans la voie d'extrusion par la poussée de la première matière plastique et en réunissant simultanément les matières plastiques,
g) extruder la première matière plastique le long de la voie d'extrusion afin de produire une partie sensiblement rigide du second élément tubulaire.
